Europäisches Patentamt

European Patent Office

Office européen des brevets

(19)

(11) Veröffentlichungsnummer: **0 013 354**
A1

(12) **EUROPÄISCHE PATENTANMELDUNG**

(21) Anmeldenummer: **79104988.5**

(22) Anmeldetag: **07.12.79**

(51) Int. Cl.³: **A 61 K 6/00**

(30) Priorität: **07.12.78 DE 2852984**

(43) Veröffentlichungstag der Anmeldung:
**23.07.80 Patentblatt 80/15**

(84) Benannte Vertragsstaaten:
**AT BE CH FR GB IT NL SE**

(71) Anmelder: **Etablissement Dentaire IVOCLAR**

**FL-9494 Schaan(LI)**

(72) Erfinder: **Dreyer-Jorgensen, Kurt, Prof. Dr.**
**Jagtvej 160**
**Kopenhagen(DK)**

(74) Vertreter: **Splanemann, Rainer et al,**
**Patentanwälte R. Splanemann Dr. B. Reitzner Tal 13**
**D-8000 München 2(DE)**

(54) **Verfahren zur Herstellung von gleichmässigen Kunststoffüberzügen auf Arbeitsmodellen für die Zahntechnik.**

(57) Verfahren zur Herstellung von gleichmäßigen Kunststoffüberzügen auf Arbeitsmodellen für die Zahntechnik, bei dem zunächst auf oder in das Arbeitsmodell ein Katalysator aufoder eingebracht wird. Dann wird auf das so behandelte Arbeitsmodell ein polymerisierbarer Kunststoff, enthaltend einen zweiten Katalysator, der in der Lage ist, zusammen mit dem ersten Katalysator den polymerisierbaren Kunststoff auszuhärten, aufgebracht. Der polymerisierte Kunststoff wird schließlich in Form einer dünnen Schicht aushärten gelassen.

EP 0 013 354 A1

Croydon Printing Company Ltd.

Die Erfindung betrifft ein Verfahren zur Herstellung von gleichmäßigen Kunststoffüberzügen auf Arbeitsmodellen für die Zahntechnik, insbesondere auf Zahnstümpfen oder Kavitäten für Inlays.

Die Herstellung einer Gußkrone beginnt mit der Präparation des Stumpfes im Mund, anschließender Abdrucknahme und Ausgießen des Abdrucks mit geeigneten Werkstoffen, z.B. Zementen, Kunststoffen, Gipsen, Metallen, keramischen Massen usw.. Das so geschaffene Arbeitsmodell (Stumpfmodell) entspricht genau der Präparation des Zahnarztes, und der Zahntechniker nimmt dann die weitere Bearbeitung vor. Auf das Arbeitsmodell wird aus Wachs eine Schicht modelliert, die dann abgezogen und in eine feuerfeste Masse eingebettet wird. Die fertige Gußkrone erhält man durch Ausgießen mit dafür geeigneten Dentallegierungen.

Das Einsetzen der Krone im Mund ist mit Schwierigkeiten verbunden, die aus den geometrischen Verhältnissen resultieren. Zur Befestigung auf dem zugeschliffenen Stumpf im Mund wird z.B. ein Phosphat- oder Carboxylat-Zement verwendet, wobei die kleinste erreichbare Schichtdicke der Zementschicht aufgrund der Korngröße des Pulvers zwischen 5 und 30 µm liegt. Da der zugeschliffene Zahnstumpf konisch ist und die Krone dazu parallele Flächen aufweist, entsteht bei zu genauer Passung Krone - Stumpf oder bei zu kleiner Krone eine okklusale Diskrepanz, d.h. in Abhängigkeit von der Filmdicke des Zements und dem Neigungswinkel des Stumpfes wird die Krone in Richtung Okklusionsfläche hin angehoben. Deshalb muß für die Zementschicht von Anfang an der notwendige Platz geschaffen werden, d.h. die Passung muß lose sein. Dies wird mit Lacken erreicht, die auf das Arbeitsmodell aufgepinselt werden, bevor man die Krone aus dem Wachs modelliert. Man verwendet aber auch expandierende Materia-

lien zum Ausgießen der Abdrücke, und es sind auch andere Verfahren zum Aufbringen einer Platzhalterschicht bekannt.

Alle genannten Verfahren haben den Nachteil, daß mit ihnen keine völlig gleichmäßige Filmdicke der Platzhalterschicht erzielt werden kann. Besonders problematisch sind die aufgepinselten Lacke. Hier schwankt die Filmdicke extrem, und die Kanten und Ecken der Präparation werden außerdem so stark abgerundet, daß beim Einzementieren der nach diesen Modellen angefertigten Kronen die genannten Verschiebungen in Richtung Okklusionsfläche und andere Ungenauigkeiten auftreten. Die ungleiche Filmdicke ist letztlich auch verantwortlich für die ungenügende Paßgenauigkeit, verbunden mit einem verschlechterten Randschluß. Die Flüssigkeit der Mundhöhle kann deshalb den zum Einzementieren verwendeten Zement leichter anlösen, wodurch das Entstehen von Sekundärkaries begünstigt wird. Die gleichen Probleme treten auch bei der Herstellung von Inlays auf.

Aufgabe der Erfindung ist es deshalb, die genannten Nachteile zu vermeiden und Kunststoffüberzüge mit einer gleichmäßigen Filmdicke zu erzeugen, um die Paßgenauigkeit und den festen Sitz der Zahnrestaurationen zu verbessern.

Gegenstand der Erfindung ist daher ein Verfahren der eingangs definierten Gattung, das dadurch gekennzeichnet ist, daß man auf oder in das Arbeitsmodell einen Katalysator auf- oder einbringt, auf das so behandelte Arbeitsmodell einen polymerisierbaren Kunststoff, enthaltend einen zweiten Katalysator, der in der Lage ist, zusammen mit dem ersten Katalysator den polymerisierbaren Kunststoff auszuhärten, aufbringt und den polymerisierbaren Kunststoff in Form einer dünnen Schicht aushärten läßt. Die nach diesem Verfahren erzielten Schichtdicken können bis zu 100 μm stark sein; vorzugsweise werden Schichten im Bereich von 5 - 50 µm, meistens aber nur von 5 - 30 µm erzeugt.

Der erste Katalysator kann in beliebiger Weise auf oder in das Arbeitsmodell auf- bzw. eingebracht werden. Vorzugsweise erfolgt dies durch Tauchen, Pinseln, Sprühen oder Auftropfen. Der erste Katalysator kann zu diesem Zweck in einer flüssigen Trägersubstanz dispergiert werden. Vorzugsweise wird er in einem Lösungsmittel gelöst und in Form einer Lösung auf das Arbeitsmodell aufgebracht. Nachdem das Lösungsmittel verdampft ist, bleibt eine gleichmäßige Schicht des ersten Katalysators auf dem Arbeitsmodell zurück. Der erste Katalysator kann aber auch direkt dem Material des Arbeitsmodells zugesetzt werden, wobei er ebenfalls in einem geeigneten Trägermedium gelöst oder dispergiert sein kann.

Die Kunststoffschicht, die nachstehend auch als Modellierschicht bezeichnet wird, besteht vorzugsweise aus einem polymerisierbaren Kunststoff, dem vor der Härtung der zweite Katalysator zugesetzt wurde. Die Modellierschicht kann nach dem Aufbringen des ersten Katalysators auf das Arbeitsmodell in ähnlicher Weise wie dieser aufgetragen werden. Vorzugsweise wird die Modellierschicht durch Tauchen aufgetragen. Verfahren, die ein gleichmäßiges Aufbringen des polymerisierbaren Kunststoffes gewährleisten, werden bevorzugt. Die Dicke der erzeugten Kunststoffschicht hängt ab von der Konzentration der verwendeten Katalysatoren, von der Art dieser Katalysatoren sowie von der Zeit, in der die beiden Katalysatoren aufeinander einwirken können.

Nach einer bevorzugten Ausführungsform der Erfindung entfernt man überschüssigen, nicht ausgehärteten bzw. nicht polymerisierten Kunststoff mit einem Lösungsmittel, z.B. mit Alkohol, und bringt die hinterbleibende Kunststoffschicht nochmals mit dem ersten Katalysator in Berührung. Dieser liegt wiederum vorzugsweise in Form einer Lösung vor, die durch Tauchen, Pinseln, Sprühen oder Auftropfen auf die hinterbleibende Kunststoffschicht aufgebracht werden kann. Auf diese Weise erzielt man neben einer vollständigen Durchhärtung der Platzhalterschicht eine vollkommen gleichmäßige Filmdicke.

Der dem erfindungsgemäßen Verfahren zugrundeliegende Effekt kann dadurch erklärt werden, daß der erste Katalysator von der Oberfläche des Arbeitsmodells aus in die Schicht aus dem polymerisierbaren Kunststoff diffundiert und zusammen mit dem zweiten Katalysator sofort die Polymerisation einer dünnen Schicht bewirkt. Da die Diffusion des ersten Katalysators aus der Oberfläche des Arbeitsmodells gleichmäßig erfolgt und unabhängig von den Abmessungen und der Gestalt des Arbeitsmodells ist, wird eine gleichmäßige Filmdicke erzeugt. Auch an den Kanten und in den Ecken ist die Filmdicke nahezu konstant.

Zur Herstellung der Modellierschicht kommen polymerisierbare Kunststoffe, die vorzugsweise kalthärtend sind, infrage, wie sie in der Dentaltechnik verwendet werden, vorzugsweise Acrylate bzw. Methacrylate. Als besonders geeignet hat sich das Reaktionsprodukt aus Bisphenol A und Glycidylmethacrylat, nachstehend Bis-GMA genannt, erwiesen, das zusammen mit anderen verdünnenden Monomeren, z.B. auf Acrylat bzw. Methacrylatbasis, oder zusammen mit nicht polymerisierenden Lösungs- bzw. Verdünnungsmitteln verwendet werden kann. Weitere geeignete Monomere zur Herstellung der Kunststoffschicht sind die mono-, di- und mehrfunktionellen Acrylate bzw. Methacrylate z.B. Methylmethacrylat, Äthylenglykoldimethacrylat, Triäthylenglykoldimethacrylat, Trimethylolpropantrimethacrylat. Auch die Urethanacrylate bzw. -methacrylate können mit Erfolg eingesetzt werden. Hierbei

0013354

handelt es sich z.B. um Umsetzungsprodukte von organischen Isocyanaten mit polymerisierbaren Acrylsäureestern, die mindestens eine Hydroxygruppe aufweisen. Beispiele für diese Acrylate sind Hydroxyäthyl- bzw. Hydroxypropylacrylate und -methacrylate, während als Isocyanat z.B. Toluylen- bzw. Hexamethylendiisocyanat Verwendung finden kann. Die polymerisierbaren Acrylate bzw. Methacrylate können allein oder als Gemische verwendet werden. Dies richtet sich sowohl nach der Viskosität des Monomers als auch nach den gewählten Maßnahmen zum Auftragen der Monomere.

Das Verfahren ist für alle Monomere bzw. deren Gemische geeignet, die polymerisierbar sind und die als solche bzw. in Form von Lösungen eine Viskosität von nicht mehr als etwa 3 Pa · s (bei 20$^o$C) haben. Vorzugsweise soll die Viskosität dieser Monomeren, Monomerengemische bzw. Lösungen 2 Pa · s nicht überschreiten, wobei ein besonders geeigneter oberer Grenzwert bei etwa 1 Pa · s liegt.

Als Werkstoffe zur Herstellung von Arbeitsmodellen eignen sich insbesondere die verschiedenen Gipsarten, wie Spezialhartgips oder Hartgips, aber auch Kunststoffe, Zemente oder leichtschmelzende Dentallegierungen können vorteilhaft eingesetzt werden. Es ist jeder Modellwerkstoff brauchbar, der an seiner Oberfläche den ersten Katalysator aufnehmen kann und nicht mit diesem reagiert. Die Haftung des ersten Katalysators auf dem Arbeitsmodell kann auch durch Zusatz eines Bindemittels zu der Katalysatorlösung verbessert werden.

Zum Polymerisieren von kalthärtenden Kunststoffen verwendet man in den Dentaltechnik sogenannte Redoxsysteme. Vorwiegend werden organische Peroxid-Polymerisationskatalysatoren verwendet. Mit

0013354

Hilfe eines zweiten Katalysators, des Aktivators, wird eine schnelle Zersetzung des Peroxids bewirkt, wodurch freie Radikale gebildet werden, die die Polymerisation in Gang bringen. Die gebräuchslichsten Peroxidkatalysatoren sind Benzolperoxid, 2,4-Dichlorbenzoylperoxid, 4-Chlorbenzoylperoxid, Laurylperoxid, 6-Butylperoxid, Methyläthylketonperoxid, Cyclohexanonperoxid. Andere Katalysatoren bestehen aus einer Kombination von Peroxid, einer Sulfinsäure oder einem Sulfinsäurederivat und Spuren von Kupfersalzen. Die Katalysatoren werden vorzugsweise in Mengen von nicht mehr als etwa 20 Gew.-% verwendet. Bevorzugte Bereiche liegen zwischen 10 und 20 Gew.-%, insbesondere zwischen 1 und 10 Gew.-%, bezogen auf das Gewicht der jeweiligen Trägersubstanz (Lösungs- bzw. Dispergiermittel).

Als zweiter Katalysator wird zur Aktivierung vorzugsweise ein Amin, z.B. ein N,N-Dialkylanilin oder ein N,N-Dialkyltoluidin verwendet. Bevorzugte Verbindungen sind N, N-Dimethyl-p-toluidin, N,N-di-(2-Hydroxyäthyl)-p-toluidin, Diäthylanilin, N,N-Dimethylanilin. Auch Kobaltverbindungen sind geeignet. Diese werden so eingesetzt, daß im Kunststoff 0.01 - 1 Gew.-% Kobalt (berechnet als Metall) enthalten ist. Ein weiterer Metallkatalysator ist ein Vanadiumsalz (z.B. Vanadyltosylat), das mit Perestern, insbesondere mit tert.-Butylperoctoat verwendet wird. Die Katalysatoren werden vorzugsweise in Mengen von nicht mehr als etwa 20 Gew.-% eingesetzt, wobei bevorzugte Bereiche zwischen 5 und 10 Gew.-%, insbesondere zwischen 0,5 und 5 Gew.-% liegen. Die angegebenen Mengen sind wiederum auf das Gewicht der Trägersubstanz, in diesem Fall des polymerisierbaren Monomeren oder Monomerengemisches bzw. der Lösung des Monomeren bzw. Monomerengemisches, bezogen.

In der Praxis müssen die beiden Katalysatortypen getrennt gelagert werden, da sonst sofort eine Polymerisation eintreten würde. Die Wechselwirkung zwischen den beiden Katalysatoren tritt erfindungsgemäß in einer dünnen Kunststoffschicht auf der Oberfläche eines Arbeitsmodells ein. Es ist im Prinzip gleichgültig, ob z.B. das Peroxid auf der Oberfläche des Arbeitsmodells sitzt; es muß aber dann dafür gesorgt werden, daß das Amin im polymerisierbaren Kunststoff enthalten ist und umgekehrt. Es können selbstverständlich im Prinzip auch andere Katalysatoren verwendet werden.

Der erste Katalysator wird vorzugsweise auf die Oberfläche des Arbeitsmodells aufgebracht. Dies kann mit und ohne Lösungsmittel, wie Aceton, Alkohol, Chloroform oder Methylenchlorid geschehen. Falls erforderlich, können auch Bindemittel für den Katalysator verwendet werden, die an glatten Oberflächen gut haften. Ferner kann das Modellmaterial auch mit einem Katalysator angerührt werden, der allerdings mit dem Modellmaterial nicht reagieren darf, da anderenfalls eine Diffusion von der Oberfläche des Arbeitsmodells in den Kunststoff nicht möglich ist. Vorzugsweise werden die organischen Peroxide in einem Lösungsmittel gelöst, und die Lösung wird auf das Arbeitsmodell aufgebracht. Das Amin ist dann im polymerisierbaren Kunststoff bzw. in einer Lösung desselben enthalten.

Die Erfindung ist durch die nachstehenden Beispiele in nicht einschränkender Weise erläutert.

### Beispiel 1

Man stellt eine Lösung aus 90 Gew.-% Aceton und 10 Gew.-% Benzoylperoxid her. Als Arbeitsmodell verwendet man ein Zahnstumpfmodell, das aus Hartgips besteht, der im Vakuum angerührt wurde. Das Stumpfmodell wird etwa 15 sec. in die Benzoylperoxid-Acetonlösung (erster Katalysator) eingetaucht und 5 min. an der Luft getrocknet. Man stellt eine

0013354

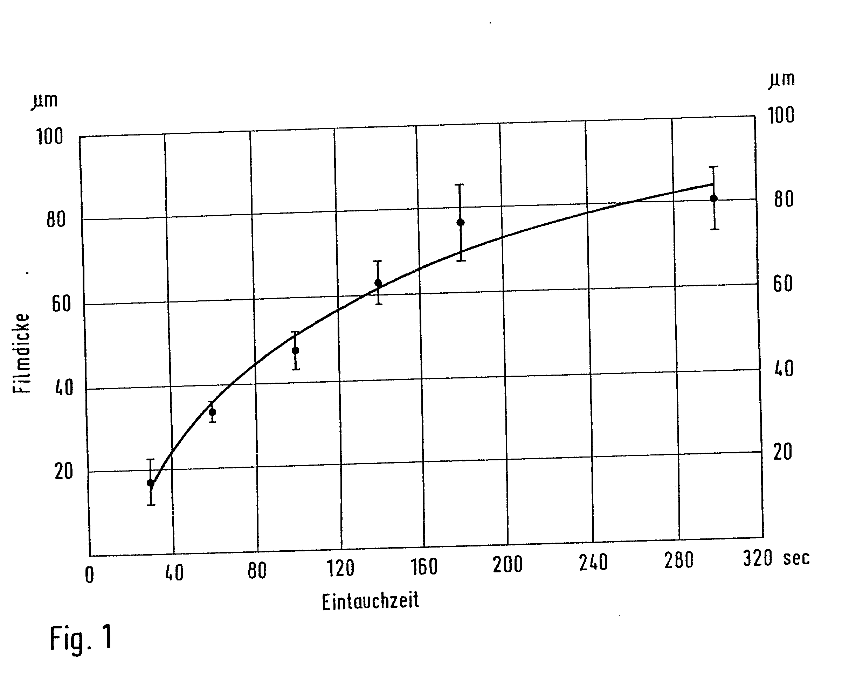

Monomermischung her, die aus 47 Gew.-% Bis-GMA und 51 Gew.-% Triäthylenglykoldimethacrylat besteht und die ferner 2 Gew.-% N,N-Diäthanol-p-toluidin als zweiten Katalysator enthält, und taucht das behandelte Stumpfmodell darin ein. Nach einer gewissen Eintauchzeit wischt man das Modell mit Aceton ab. Man erhält einen gleichmäßigen dünnen Überzug. Die Filmdicke wurde mit einem Mikroskop gemessen. Es wurden jeweils 9 Meßpunkte ausgewertet. Die Abhängigkeit der Filmdicke von der Eintauchzeit des Stumpfmodells in die Monomermischung zeigt Fig. 1.

Zum Vergleich wurde auf das Arbeitsmodell kein Katalysator aufgetragen, sondern derselben Mischung aus Monomer und Amin wurde 1,0 Gew.-% Benzoylperoxid zugesetzt; diese Mischung wurde dann mit einem Pinsel auf das Stumpfmodell aufgetragen. Messungen ergaben eine unterschiedliche Filmdicke, vor allem an den Kanten und in den Ecken.

## Beispiel 2

Man stellt eine 10 Gew.-%ige Benzoylperoxidlösung in Chloroform her (erster Katalysator) und sprüht die Lösung mit einem Zerstäuber auf ein Stumpfmodell aus Gips. Das Monomerengemisch enthält 59 Gew.-% Bis-GMA, 19 Gew.-% Triäthylenglykoldimethacrylat und 20 Gew.-% Bisphenol A-dimethacrylat, dem 2 Gew.-% N,N-Dimethyl-p-toluidin (zweiter Katalysator) zugesetzt sind. Das Monomergemisch wird ebenfalls mit einem Zerstäuber gleichmäßig, aber im Überschuß, auf das Stumpfmodell aufgesprüht. Das behandelte Stumpfmodell wird bei etwa 22°C 3 Minuten stehengelassen. Es zeigt sich, daß auch nach dieser Zeit das überschüssige Monomer bis auf einen dünnen Film nicht polymerisiert ist; es wird mit Äthanol abgewaschen. Die Dickenmessung der Filmschicht ergab 75 μm; es waren keine signifikanten Schwankungen festzustellen.

## Beispiel 3

Die Arbeitsweisen nach den Beispielen 1 und 2 werden mit der Abweichung wiederholt, daß anstelle der Peroxidlösung (erster Katalysator) eine 2%ige Aminlösung (N,N-Dimethyl-p-toluidin) auf das Arbeitsmodell aufgebracht wird. Das Amin im Monomergemisch wird durch 2,5 Gew.-% Benzoylperoxid ersetzt. Es ergeben sich äquivalente Schichtdicken.

## Beispiel 4

Ein Stumpfmodell wird aus einer Wismut-Zinnlegierung hergestellt. Man löst Nitrocellulose in Äthylacetat auf und setzt der Lösung 10 Gew.-% Benzoylperoxid zu. Das Stumpfmodell wird 30 sec. in die Lösung getaucht und anschließend getrocknet. Das so behandelte Stumpfmodell taucht man 20 sec in Äthylenglykoldimethacrylat, dem 5 Gew.-% Diäthanol-p-toluidin zugesetzt sind. Nach 10 min. wischt man überschüssiges, nicht polymerisiertes Monomer mit Äthanol ab und taucht das Stumpfmodell 10 sec. in die Peroxidlösung nach Beispiel 1. Nach 20 min. wird die Schichtdicke gemessen. Man erhält einen sehr harten, gleichmäßigen dünnen Film von etwa 30 µm.

## Beispiel 5

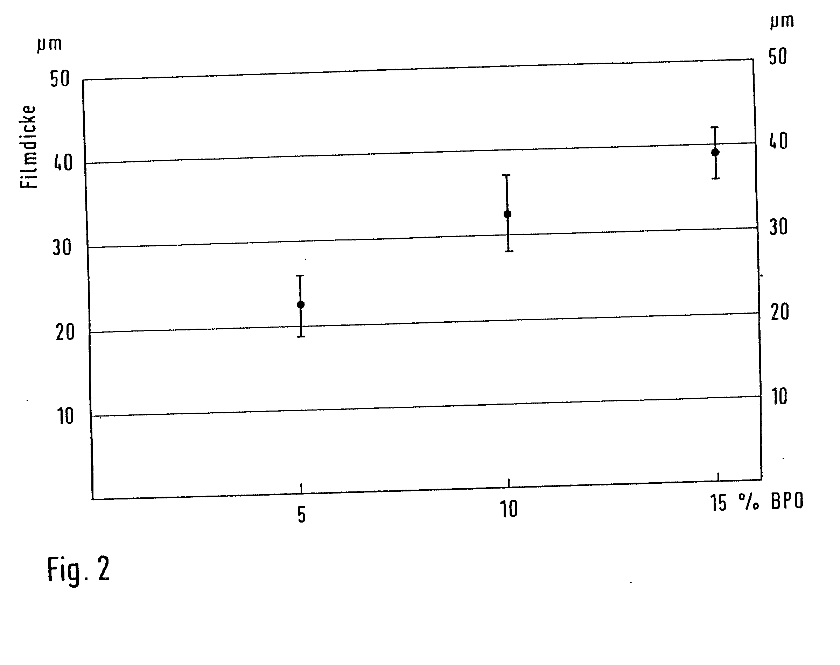

Die Arbeitsweise von Beispiel 1 wird mit der Abweichung wiederholt, daß die Konzentration der Benzoylperoxidlösung variiert wird. Es zeigt sich eine gewisse Abhängigkeit der Filmdicke von der Peroxid-Konzentration (vgl. Fig. 2).

## Beispiel 6

Das Modell für ein Inlay besteht aus Hartgips. Man verwendet die im Beispiel 2 erwähnte, 10 gew.-%ige Benzoylperoxidlösung, mit der die Kavitätenwände des Modells ausgepinselt werden.

Das Monomergemisch besteht aus 10 Gew.-% Methylmethacrylat, 40 Gew.-% Bis-GMA, 45 Gew.-% Triäthylenglykoldimethacrylat und 5 Gew.-% Dimethyl-p-toluidin. Das Modell wird 20 sec. in das Monomerengemisch eingetaucht, dann entfernt und mit Methanol abgewaschen. Die gemessene Filmschichtdicke war gleichmäßig stark. Vor allem aber waren die Rundungen zwischen Kavitätenwand und Kavitätenboden weniger stark ausgeprägt als bei Vergleichsmodellen, die nach der herkömmlichen Methode hergestellt wurden.

## Beispiel 7

Ein Stumpfmodell aus Chemiegips wird 30 sec. in eine 2%ige Benzoylperoxid-Acetonlösung eingetaucht. Der Stumpf wird getrocknet und 20 sec. in Triäthylenglykoldimethacrylat getaucht, dem 2 % Dimethyl-p-toluidin zugesetzt sind. Danach wird der Stumpf 1 min in der Peroxid-Acetonlösung gespült.

Der sehr harte Film hat eine gleichmäßige Schichtdicke.

## Beispiel 8

Das Stumpfmodell wird aus einem Polyesterharz (Roskydal A 500 d.Fa. Bayer) hergestellt. Man stellt eine 18 %ige Nitrocellulose-Äthylacetatlösung her und gibt 5 % Benzoylperoxid hinzu. Dieses Gemisch wird mit einem Pinsel auf das Stumpfmodell aufgetragen und 10 min. getrocknet. Man taucht das Modell in ein Gemisch aus 97 % Äthylenglykoldimethacrylat und 3 % Dimethyl-p-toluidin etwa 30 sec. ein und spült mit Aceton nach.

Die Schichtdicke des Films ist gleichmäßig.

## Beispiel 9

Die Arbeitsweise von Beispiel 8 wird mit der Abweichung wiederholt, daß jeweils das Benzoylperoxid gegen Dimethyl-p-toluidin ausgetauscht wird. Die gemessenen Schichtdicken des Films sind gleichmäßig.

- 1 -

001335⁄

Etablissement Dentaire
I V O C L A R

FL-9494  Schaan

7. Dezember 1979

u.Z.: 2657-X-10.833

Europäische Patentanmeldung

Verfahren zur Herstellung von gleichmäßigen
Kunststoffüberzügen auf Arbeitsmodellen für
die Zahntechnik

P A T E N T A N S P R Ü C H E

1.  Verfahren zur Herstellung von gleichmäßigen Kunststoffüberzügen auf Arbeitsmodellen für die Zahntechnik,
dadurch gekennzeichnet, daß man auf oder in das Arbeitsmodell einen Katalysator auf- oder einbringt, auf das
so behandelte Arbeitsmodell einen polymerisierbaren Kunststoff, enthaltend einen zweiten Katalysator, der in der
Lage ist, zusammen mit dem ersten Katalysator den polymerisierbaren Kunststoff auszuhärten, aufbringt und den
polymerisierbaren Kunststoff in Form einer dünnen Schicht
aushärten läßt.

2.  Verfahren nach Anspruch 1, dadurch gekennzeichnet,
daß man den ersten Katalysator durch Tauchen, Pinseln,
Sprühen oder Auftropfen auf bzw. in das Arbeitsmodell
auf- bzw. einbringt.

3. Verfahren nach Anspruch 1 oder 2, dadurch gekennzeichnet, daß man den ersten Katalysator in Form einer Lösung auf das Arbeitsmodell aufbringt.

4. Verfahren nach Anspruch 1 oder 2, dadurch gekennzeichnet, daß man den ersten Katalysator direkt dem Material des Arbeitsmodells zusetzt.

5. Verfahren nach einem der Ansprüche 1 bis 4, dadurch gekennzeichnet, daß man als ersten Katalysator ein organisches Peroxid und als zweiten Katalysator ein Amin verwendet oder umgekehrt.

6. Verfahren nach einem der Ansprüche 1 bis 5, dadurch gekennzeichnet, daß man einen polymerisierbaren Kunststoff bzw. eine Lösung desselben mit einer Viskosität von nicht mehr als etwa 3 Pa · s verwendet.

7. Verfahren nach einem der Ansprüche 1 bis 6, dadurch gekennzeichnet, daß man nicht ausgehärteten Kunststoff mit einem Lösungsmittel entfernt.

8. Verfahren nach einem der Ansprüche 1 bis 7, dadurch gekennzeichnet, daß man die Katalysatoren in Mengen von nicht mehr als etwa 20 Gew.-%, bezogen auf das Gewicht der jeweiligen Trägersubstanz, verwendet.

9. Verfahren nach einem der Ansprüche 1 bis 8, dadurch gekennzeichnet, daß man nicht ausgehärteten Kunststoff mit einem Lösungsmittel entfernt und die hinterbleibende Kunststoffschicht nochmals mit dem ersten Katalysator in Berührung bringt.

Fig. 1

Fig. 2

0013354

Europäisches
Patentamt

EUROPÄISCHER RECHERCHENBERICHT

Nummer der Anmeldung

EP 79 10 4988

| Kategorie | Kennzeichnung des Dokuments mit Angabe, soweit erforderlich, der maßgeblichen Teile | betrifft Anspruch |
|---|---|---|
| X | AT - B - 320 838 (ET. RIVOLAN)<br><br>* Seite 2, Zeile 60 - Seite 3, Zeile 33; Beispiele; Ansprüche *<br><br>& (Technisch Übereinstimmende):<br><br>GB - A - 1 281 957<br><br>FR - A - 2 094 493<br><br>NL - A - 70 15428<br><br>---- | 1-3,5, 8,9 |

**EINSCHLÄGIGE DOKUMENTE**

**KLASSIFIKATION DER ANMELDUNG (Int.Cl.³)**

A 61 K 6/00

**RECHERCHIERTE SACHGEBIETE (Int. Cl.³)**

A 61 K 6/00
6/08
6/10

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 20-03-1980 | WILLEKENS |

EPA form 1503.1 06.78